# EUROPEAN PATENT SPECIFICATION

(11) **EP 1 352 248 B1**
(45) Date of publication and mention of the grant of the patent: **11.02.2009**
(21) Application number: 02716273.4
(22) Date of filing: 18.01.2002
(51) Int. Cl.: G01N 33/68

(54) **TEST FOR TRANSMISSIBLE SPONGIFORM ENCEPHALOPATHIES**
TEST FÜR ÜBERTRAGBARE SPONGIFORME ENCEPHALOPATHIEN
TEST POUR ENCEPHALOPATHIES SPONGIFORMES TRANSMISSIBLES

(30) Priority: 19.01.2001 IE 20010042
(43) Date of publication of application: 15.10.2003
(73) Proprietor: ENFER TECHNOLOGY LIMITED, Dublin 2 (IE)
(72) Inventor: O'CONNOR, Michael, County Dublin (IE)
(74) Representative: Gates, Marie Christina Esther
(86) International application number: PCT/IE2002/000006
(87) International publication number: WO 2002/057789

(56) References cited:
- EP-A- 0 861 900
- WO-A-98/35236
- DE-A- 3 921 839
- US-A- 5 633 349
- US-A- 5 773 572

## Description

The present invention relates to a method of detecting Transmissable Spongiform Encephalopathies, and in particular to a test which can be conducted on living or dead animals or humans to identify TSEs.

### Background to the invention

Spongiform Encephalopathies are a group of degenerative neurological diseases. There are a number of examples of Spongiform Encephalopathies including BSE (Bovine Spongiform Encephalopathy), Scrapie, Creutzfeldt-Jacob Disease (CJD) Gerstmann-Straussler--Scheinker Syndrome, Kuru, Transmissible Mink Encephalopathy, Chronic Wasting Disease of Mule Deer, Feline Spongiform Encephalopathies and other Spongiform Encephalopathies found in animals such as elk, naya, greater kudu, gemsbok and tigers. It has also been reported that BSE can be transmitted under laboratory conditions to mice and pigs. This crossing of species barriers by the infective agent has led to increased concern that transfer to humans could occur.

Bovine spongiform Encephalopathy (BSE) is a degenerative brain disorder of cattle which is popularly known as "mad cow disease". It has a slow incubation period, up to four or five years with symptoms of progressive degeneration of the mental state in cows include loss of co-ordination and staggering gait, lack of interest in their surroundings, disinterest in feed and water, or unpredictable behaviour, including aggressiveness. Affected cattle show symptoms when they are three to ten years old.

First identified in Great Britain in November 1986, over 100,000 cases have since been recorded there. Post mortems of affected cattle reveal a characteristic pattern of vacuolation in the brain tissue due to destruction of neural cells and the deposition of unusual protein fibres, that give the brain a spongy (spongiform) texture. Similar spongiform diseases have been recognised in humans (for example, Creutzfeldt-Jakob disease or (CJD) for over a century and in sheep (scrapie) for over 200 years. The agent thought to be responsible for Transmissable Spongiform Encephalopathies is an infective protein known as a prion. The prion is an infective particle comprising protein only and no nucleic acid, the presence of nucleic acid being required in the case of a conventional virus. In Scrapie in particular one protein known as prion protein or Prp^{sc} has been found to co-purify with infectivity and can produce a Scrapie-like condition in brain cell cultures from other animals, such as hamsters under laboratory conditions. Prp^{sc} is the only known component of the characteristic protein fibres deposited in the brain tissue of Scrapie-infected sheep. This protein, the PrP^{sc} appears to undergo a structural modification, whereas the term PrP^{c} is used in respect of the normal cellular counterpart of PrP^{sc}. The natural function of PrP^{c} is not known, but it appears likely that it has an essential structure or functional role in the organism.

Recycled animal tissue, which had been routinely fed to British dairy cows as a protein supplement has been identified as the source of the infection. It is believed that BSE was originally spread from sheep's brains infected with scrapie and that its spread was accidentally accelerated by the ingestion of brain tissue taken from cows that had become infected with BSE. Therefore, the British Government introduced compulsory destruction of suspect animals and their carcasses beginning in 1988. The feeding of animal tissue to cows was banned in Britain in July 1988.

Since the initial report of the disease, consumers have feared that it might be transferable to humans through milk or beef products, particularly since Kuru, a related disease is known to be spread by ritualistic cannibalism among New Guinea tribesmen. In late 1990, consumer concern over the transmission of BSE to humans triggered a collapse in the beef market. A similar scare struck Germany in mid-1994.

In 1996 ten cases of a newly described type of fatal CJD (Variant CJD) were identified. The victims had distinct brain tissue symptoms, were all under the age of 42, and had no hereditary record of the disease. It has been suggested that the victims may have contacted the disease through contact with BSE-infected cattle before the eradication of suspected animals had taken effect. The identification of Variant CJD led to a dramatic drop in beef consumption in Britain, and the banning of British and in some instances Irish beef imports in various countries worldwide.

US5773572 discloses synthetic polypeptides having at least one antigenic site of a prion protein together with methods for their use and manufacture and antibodies raised against such polypeptides. Diagnostic kits using the polypeptides and/or antibodies are also disclosed. EP0861900 discloses monoclonal antibodies useful in sensitive and specific immunological assays for the identification of prions in various tissues and body fluids, the production of such monoclonal antibodies by means of immunisation of PrP<0/0> mice by means of a new recombinant fragment of PrP and the use of the antibodies, e. g. for therapeutic and preventive treatments of humans and animals suffering from prion diseases.

Therefore, for both veterinary and economic reasons, there is an urgent need to provide a method for diagnosis and a diagnostic kit to detect infection with BSE, Scrapie, Variant CJD and other related spongiform Encephalopathies, in livestock, animal carcasses and meat generally, as well as in pharmaceuticals and humans.

### Objet of the invention

It is thus an object of the present invention to provide a method of testing animals, pharmaceuticals and humans for the infective agent responsible for TSE. It is also an object to provide a multitissue test system which can be used on both blood or blood derived products and solid tissue. It is a further object that the method be rapid, with the result being available in a matter of hours, and that it should be cheap, reliable and user friendly.

Until this time there has been no method of detecting TSE which could be conducted on blood or blood derivatives. Up to now the only method of determining whether animals were suffering from TSE or human beings were suffering from a related disease was to carry out a pathological post mortem examination of brain tissue. Thus a test which could be tested on blood has the enormous advantage that it can be conducted on living animals or human beings. This gives rise to the possibility that infected animals could be removed from the population and slaughtered, in an attempt to prevent the spread of infection to other animals in the population. It would also mean that infected human beings could be identified, with the possibility that medical treatment could be administered and a possible cure could be found for the disease. The test would also prevent the entry of infected meat into the human food chain, thus reducing the possibility of humans contracting variant CJD or other related diseases which may be transmitted by eating infected meat. It would also restore consumer confidence in meat or meat-derived products, which would be advantageous to both the farming community and the meat industry in general.

It has now surprisingly been found that a test for TSEs can be carried out on blood or blood derived products. The test has the advantage that it can also be carried out on solid tissue samples.

### Summary of the Invention

According to the present invention there is provided a method of detecting TSE comprising (a) treating a sample of tissue, blood or a blood derivative from a test subject with a homogenization buffer comprising alcohol in an amount up to 30% and between 5% and 30% detergent and dispensing the sample onto an inert support (b) adding an agent which degrades normal prion protein (c) adding an agent which denatures abnormal prion protein, (d) adding a prion - specific antibody and (e) detecting binding of the antibody to the sample.

Step (a) may further comprise the addition of a protein, preferably a high molecular weight protein such as bovine serum albumin, ovalbumin, normal serum of animal origin, an albumin substitute. The protein may be formulated in a buffer containing up to 5%, preferably 0.05 to 0.4% protein.

Preferably the alcohol used in step (a) is a C₁ to C₂₄ alcohol such as methanol, ethanol, propanol, buntanol, pentanol, hexanol, heptanol, octanol, nonanol, decanol, undecanol, dodecanol, tridecanol, tetradecanol, pantadecanol, cetyl alcohol, heptadecanol, stearyl alcohol, nonadecanol, eicosanol, docasanol, dodecyl alcohol, lauryl alcohol, octadecanol, and derivatives of each, including primary, secondary and tertiary derivatives, or the like. More preferably the alcohol is a C₁ to C₅ alcohol, most preferably methanol/ethanol. The alcohol may be formulated in a buffer containing up to 30% (by weight), preferably 10 to 20% more preferably 8 to 12% alcohol.

Preferably the detergent of step (a) is selected from sodium dodecyl sulphate, sarcosyl, lithium dodecyl sulphate, cholic acid, deoxycholic acid, Triton X-100, octanoic acid, Brij (trade mark) 35 solution, N-Cetyl-N,N,N-trimethylammoniumbromide, CHAPS (3-[(-cholamidopropyl)-dimethylammonio]-propane-sulphonate), CHAPSO (3-[(-cholamidopropyl)-dimethylammonio]-hydroxypropane-sulphonate), Digitonin, nonanoyl-N-methylglucamide, n-Octyl-B-D-glucopyranoside, alkyl sodium sulphate, dioctyl sodium sulfosuccinate, sodium oleate, sodium lauryl sulphate, 1,2,3-Octanetriol, 3-Diethylamino-1 propyne, Chondroitinase ABC (available from Fluka), sodium Ionophore 1 (available from Fluka), most preferably sodium dodecyl sulphate. The detergent may be formulated in a buffer containing 5 to 30%, more preferably 10 to 20% detergent. The detergent may be an anionic detergent.

In step (b) the agent which degrades normal prion protein may suitably be a protease, suitably a broad spectrum protease. Preferably the protease is selected from proteinase K, subtilisin Carlsberg, pepsin and derivatives of each or the like. The protease may be formulated in a buffer containing up to 10mg/ml, preferably 0.05mg/ml to 4mg/ml, more preferably 0.2mg/ml to 0.6mg/ml protein.

Preferably the agent which denatures abnormal prion protein in step (c) is a denaturing salt solution. Suitably the agent is selected from guanidine, urea, a standard denaturing solution e.g. Merck denaturing solution (commercially available from Merck) and derivatives of each or the like. In a particularly preferred embodiment the denaturing agent may further include up to 0.5M sodium hydroxide. The denaturing salt may be formulated in a buffer containing 0.1 to 10M, preferably 2 to 8M denaturing salt.

The buffer of step (a) (homogenising buffer) may be made up to have a final pH of 5.0 to 11.0, preferably pH 7.0 to 9.0, more preferably pH 7.5 to 8.5. The buffer of step (b) (differential buffer) may be made up to a final pH of 2.0 to 11.0, preferably pH 2.0 to 8.5, more preferably 7.5 to 8.5. The buffer of step (c) (assay priming buffer) should have a pH of 1 to 14, more preferably ph 9 to 12, most preferably at least 11.

Preferably, following step (b) the reaction mixture is washed with distilled water or a first wash buffer comprising up to 10%, preferably 4 to 6% buffered solution.

The assay priming buffer is allowed to incubate with the reaction mixture for about 5 to 30 minutes, preferably 10 to 20 minutes. Suitably, the mixture is then washed with a second wash buffer having a final pH of 5.0 to 11.0, preferably pH 7.0 to 8.0.

The first wash buffer may comprise sodium chloride, calcium chloride, zinc chloride or the like. The second wash buffer may comprise phosphate buffered saline or the like with the optional addition of 0 to 5% Tween 20 or the like.

Preferably the blood or tissue sample is dispensed onto a support, suitably a microtitre tray, and may then preferably be subjected to an optional centrifuge step which serves to clarify or "clean up" the samples for further processing.

The prion specific antibody is raised to a prion sequence which is to the 3' side of amino-acid number 60 of the prion sequence.

The prion specific antibody is suitably directed against the prion peptide:-
PRION SEQUENCE N-TERM: 
MVKSHIGSWILVLFVVAMWSDVGLCKKRPKPGGGWNTGGSRYPGQ-44
GSPGGNRYPPQGGGGWGQPHGGGWGQPHGGGWGQPHGGGWGQP-87
GGGGWGQGGSHSQWNKPSKPPKTNMKHVAGAAAAGAVVGGLGGY- 131
MLGSAMSSPLIHFGNDYEDRYTRENMYRYPNQVYYRPVDRYSNQNN-177
 wherein the single letter amino acid code is an defined herein, as well as derivative and fragments of the sequences, including those that may include D-isomer amino acids as well be understood by those skilled in the art , modification of this sequnce by substitution, deletion or insertion of amino acids could result in a peptide with the same activity.

The blood sample may either be a whole blood sample, or it may be a blood derived sample such as a serum sample or a buffycoat preparation which includes white blood cells and leucocytes. If the test is used on a solid tissue sample a sample of about 0.3g to 1.5g is used.

Suitably the blood or tissue sample is homogenised. If the blood sample is gelatinous an enzyme degradation step may be included. The enzyme degradation step may include the addition of DNase, Rnase, a protease or a lipase.

The prion specific antibody may be raised to a variety of species - specific prion sequences in any species. The antibody may be for example raised in rabbits, and the immunoassay may involve the use of a secondary antibody, suitably a goat (species) anti -rabbit antibody conjugated with horseradish peroxidase.

The detection method is suitably an enhanced chemiluminesence assay, although colourimetric detection, flourimetric detection or radioimmunoassay detection could also be used.

In a particularly preferred embodiment there is provided a test for Transmissible Spongiform Encephalopathies comprising obtaining a sample of blood or a blood derivative or tissue from a test subject, reacting the tissue, blood or blood derivative sample with a prion specific antibody and detecting the binding of the antibody to the sample by standard immunoassay, wherein the tissue, blood or blood derivative sample is mixed prior to addition of the antibody with a first buffer comprising 0 to 30% of C₁ to C₅ alcohol, 5 to 30% sodium dodecyl sulphate, and 0 to 5% bovine serum albumin made up to volume in about 0.1 M TRIS/HCL or phosphate buffer, the buffer having a final pH of 7.0 to 9.0.

Preferably the blood or tissue sample is dispensed onto a support, more preferably an inert plastic support, suitably a microtitre tray, and a second buffer comprising 1 to 10 mg/ml of a broad spectrum protease enzyme in about 0.1 M TRIS/HCL or phosphate buffer with a final pH of 7.5 to 8.5, is added. The buffer is suitably diluted 1 in 5 to give a working strength of 0.2 mg/ml to 2 mg/ml. The sample is then incubated, preferably for about 10 to 180 mins at about 37 - 70°C, more preferably at 60°C.

Following incubation, the support suitably is washed with a volume of a first wash buffer comprising 0 to 10% salt solution. Preferably, a third buffer comprising either 0.5 to 3M guanidine or 2 to 8M urea, and 0.1 to 0.2M sodium hydroxide made up in reverse osmosis distilled water with a pH of at least 11 at room temperature, is added to the support. Suitably, the support and third buffer are allowed to incubate for about 15 minutes and are washed with a wash buffer comprising 0.1M phosphate buffered saline and 0.5% Tween 20^{™}.

The sample preparation is then complete and ready for immunoassay.

The invention also provides a test for Transmissable Spongiform Encephalopathies comprising homogenizing a sample of blood or blood derivative or tissue from a test subject in a homogenization buffer comprising alcohol in a amount up to 30% and between 5% and 30% detergent, reacting the tissue, blood or blood derivative sample with a prion specific antibody and detecting the binding of the antibody to the sample by standard immunoassay, wherein the blood or tissue sample is dispensed onto an inert support and incubated with a buffer comprising up to 10 mg/ml, preferably 0.05mg/ml to 4mg/ml, more preferably 0.2mg/ml to 0.6mg/ml, of a broad spectrum protease enzyme in a buffer with a final pH of 2.0 to 11.0, preferably pH 2.0 to 8.5, more preferably 7.5 to 8.5, prior to reaction with the antibody. Preferably the inert support is centrifuged following dispense of the sample and before addition of the protesase(differential buffer). The protease may be formulated in a buffer comprising 0.1M TRIS or phosphate.

In a further aspect the invention provides a test for Transmissable Spongiform Encephalopathies comprising homogenizing a sample of blood or a blood derivative or tissue from a test subject in a homogenization buffer comprising alcohol in a amount up to 30% and between 5% and 30% detergent, reacting the tissue, blood or blood derivative sample with a prion specific antibody and detecting the binding of the antibody to the sample by standard immunoassay, wherein the sample is incubated with a buffer comprising 0.1 to 10M denaturing solution, preferably 3 to 6M, more preferably 0.5 to 3M Guanidine (optimal range 2 to 4M) or 2 to 8M urea (optimal range 4 to 6M), and up to 0.5M, preferably 0.1 to 0.2M sodium hydroxide made up in reverse osmosis distilled water, with a pH of at least 11 at room temperature, prior to reaction with the antibody.

Also disclosed is a buffer selected from:
1. 0 to 30% of C₁ to C₂₄ alcohol (optimal range 10 to 20, preferably 8 to 12%) 5 to 30% detergent (optimal range 10 to 20%), and a 0 to 20% protein (optimal range 0.05 to 0.4%) made up to volume in a buffer having a final pH of 5.0 to 11.0 (optimal range 7.5 to 8.5).
2. A buffer comprising of 0.05mg/ml to 4mg/ml (optimal range 0.2mg/ml to 0.6mg/ml) of a protease enzyme in a buffer having a final pH of 2.0 to 11.0 (optimal range 2.0 to 8.5).
3. A buffer comprising 0.1 to 10M denaturing solution (optimal range 2 to 8M).

All buffers are produced and supplied by Enfer Scientific Ltd.

### Homogenising Buffer:

| **Ingredients** | **Working Range** | **Optimal Concentration** |
|---|---|---|
| Methanol or Ethanol | 0 to 30% | 16% |
| Sodium Dodecyl Sulphate | 5 to 30% | 15% |
| Bovine Serum Albumin | 0 to 5% | 0.1% |

This buffer is made up to the required volume, stirring in 0.1M TRI/HCL Buffer, final pH of 8.0 ±1.

### Differential Buffer:

| **Ingredients** | **Working Range** | **Optimal Concentration** |
|---|---|---|
| Proteinase K | 0.05 to 4mg/ml | 0.4mg/ml |

This buffer is made up to the required concentration in 0.1M TRIS/HCL Buffer, final pHof8.0±1.

### Assay Priming Buffer:

| **Ingredients** | **Working Range** | **Optimal Concentration** |
|---|---|---|
| Guanidine | 0.5 to 6M | 3M |
| Sodium Hydroxide | 0 to 0.5M | 0.15M |

### OR

| **Ingredients** | **Working Range** | **Optimal Concentration** |
|---|---|---|
| Urea | 2 to 8M | 6M |
| Sodium Hydroxide | 0 to 0.5M | 0.15M |

This buffer is made up to the required concentration in reverse osmosis distilled water and allowed to reach room temperature before checking the pH, which should be greater than 11.

### Wash Buffer1:

| **Ingredients** | **Working Range** | **Optimal Concentration** |
|---|---|---|
| Sodium Chloride | 0 to 10% | 5% |

This buffer is made up to the required volume in reverse osmosis distilled water, pH irrelevant.

### Wash Buffer 2:

| **Ingredients** | **Working Range** | **Optimal Concentration** |
|---|---|---|
| Phosphate Buffered Saline | 0 to 5M | 0.1M |
| Tween 20 | 0 to 5% | 0.5% |

This buffer is made up to the required volume in reverse osmosis distilled water, with a pH of 7.6 ± 1.

Further disclosed is a buffer selected from:
1. 0 to 20% of C₂ to C₅ alcohol, 15 to 30% sodium dodecyl sulphate, or 5 to 30% sodium dodcyl sulphate and 0 to 5% Sarcosyl, 0 to 0.5% bovine serum albumin and made up to volume in about 0.1M TRIS or phosphate buffer, the buffer having a final pH of 7.0 to 9.0;
2. A buffer comprising 1 to 10 mg/ml of a broad spectrum protease enzyme in about 0.1M TRIS or phosphate buffer with a final pH of 7.5 to 8.5;
3. A buffer comprising either 0.5 to 3M Guanidine or 2 to 8M urea, and 0.1 to 0.2M sodium hydroxide made up in reverse osmosis distilled water, with a pH of at least 11 at room temperature.

The invention will now be described in greater detail with reference to the following example.

### EXAMPLE

The requirements for the TSE immunoassay are as follows:
a) A sample of solid tissue, blood or blood derived preparation.
b) A series of preparative sample treatments.
c) A prion specific antibody.
d) Sensitive method of detection.

a) A sample of blood is taken using either an anti-coagulant coated tube or a non-coated blood tube. Two options are available for testing:
   i) Whole blood sample.
   ii) A blood derived sample - serum sample or a buffycoat (white blood cells, leucocytes etc.) preparation.
   A sample of solid tissue is taken from an animal or human by a suitable method e.g. biopsy or dissection.
b) On reaching the laboratory the sample is identified using barcodes. The barcode assigned to each sample incorporates information on the place of origin of the sample (slaughter plant, clinic, hospital), the date the sample is taken and a traceable identification sample number. An amount of each sample, is removed, weighed and placed into a sample bottle/tube/stomacher bag for homogenisation (stomaching, sonication or vortexing). If the sample is particularly gelatinous, an enzymatic degradation step can be included - addition of DNase, Rnase, protease or lipase used in accordance with supplier instructions. This sample bottle or bag containing the homogenised original sample will be assigned an identical barcode to the original sample. This allows full traceability throughout the system. A fixed volume to weight ratio (1:1 to 1:25) of Homogenising Buffer, supplied by Enfer Products, Castleblake, Rosegreen, Cashel, Co. Tipperary, is added to the sample bottle and the sample homogenised by stomaching, vortexing or sonication. If the sample is particularly gelatinous, an enzymatic degradation step can be included -addition of DNase, Rnase, protease or lipase used in accordance with supplier instructions. The sample is then dispensed (200µl) in duplicate onto a polypropylene 96 well microtitre plate. Blank, Negative and Positive controls supplied by Veterinary Research Laboratories, Abbotstown, Dublin are also added to this plate. This plate is centrifuged in a plate centrifuging rotor at 4000 rpm for 10 minutes. 20ul of Differential Buffer, supplied by Enfer Scientific, Castleblake, Rosegreen, Cashel, Co. Tipperary, is added preferably to a plastic, totally inert microtitre plate (e.g. DYNEX Microlite 1 plate made from immulon 1 plastic). The plate has to be inert for optimal completion of the assay. Use of charged plates decreases the results. 100µl of each of the centrifuged samples and controls is then transferred from the polypropylene centrifuged plate to the inert assay microlite plate containing the volume of Differential Buffer and a plate coverslip placed over the wells. The plate is then incubated at 37°C for 60 minutes, the coverslip removed and the plate washed in Wash Buffer 1, supplied by Enfer Scientific, Castleblake, Rosegreen, Cashel, Co. Tipperary. Assay priming Buffer, supplied by Enfer Scientific, Castleblake, Rosegreen, Cashel Co. Tipperary, is added to each well at a volume of 200µl. The plate is covered with a coverslip and incubated at 37°C for 15 minutes. The coverslip is removed on completion of the incubation and washed with Wash Buffer 2, supplied by Enfer Scientific, Castleblake, Rosegreen, Cashel Co. Tipperary, is then added to the samples on the plate. This step completes the sample preparation procedure.
c) The samples are now ready for immunoassay. This requires a prion specific antibody. An example for such an antibody is a rabbit anti - PrP, raised by injecting rabbits with ovalbumin conjugated synthetic prion peptides, a peptide which is licenced to Enfer Scientific by Proteus International UK. This peptide was identified by Proteus International UK using molecular modelling techniques followed by Western Blot and ELISA analysis. The peptides are synthesised in bulk using solid-phase peptide synthesis. Use of other anti-PrP antibodies raised to a variety of species - specific prion sequences in any species, will also yield satisfactory results in this assay. The peptide described herein is only one example of an antibody that has been successful in the assay. Both polyclonal and monoclonal antibodies can be used for assay. A fixed volume (150µl) of this specific antibody at optimal working strength dilution in Wash Buffer 2 is added to the microlite plate, incubated at 37°C for 40 minutes and washed using Wash Buffer 2. A fixed volume (150µl) of species-anti-rabbit(species)-HRP supplied by DAKO, USA at optimal working strength dilution in Wash Buffer 2 is added to the plate, incubated at 37°C for 30 minutes and washed using Wash Buffer 2. Detection of results is now possible.
d) Detection of results is by means of Enhanced Chemiluminescence. A fixed volume of a chemiluminescent reagent- Amerlite reagent (150µl) supplied by Johnson & Johnson, UK, is added to the microtitre plate and incubated at 37°C for 3 minutes. The light signal is read using a Labsystems Chemiluminometer, the results assigned to the corresponding barcode and a results report printed.

### DETAILED PREPARATIVE PROTOCOL

### 1. HOMOGENISATION

1.1 Blood or blood derived or tissue sample is placed into a sample bottle/tube/bag.
1.2 1 to 25, preferably 10 to 15 times the sample weight of Homogenising Buffer is added to the sample.
1.3 This mixture is homogenised (stomacher, sonicator, vortex) for 2 minutes or until the sample is totally homogenised.
1.4 Enzymatic degradation option may be applied here at the discretion of the operator.
1.5 The resultant homogenate may be filtered, if particulate matter remains in the sample.

### 2. PLATING

2.1 A V-96-well microtitre polypropylene plate is used for centrifugation of 200µl of each sample.
2.2 200µl of blank control, onto the plate, positions A1,2 of the centrifuge plate.
2.3 200µl of negative control (known negative BSE homogenate), supplied by the Veterinary Research Laboratories, Abbotstown, Castleknock, Dublin, Ireland is dispensed - 4 replicates- onto the plate, positions B 1,2 and C1,2.
2.5 200µl of positive control, (known positive BSE homogenate), supplied by the Veterinary Research Laboratories, Abbotstown, Castleknock, Dublin, Ireland is dispensed - 4 replicates- onto the plate, positions D1,2 and E1,2.
2.6 200µl of each (filtered, enzymatically treated) homogenate is dispensed in duplicate onto the plate, into the remaining positions.
2.7 The plate is centrifuged at 4000rpm for 10 minutes.
2.8 20µl of Differential Buffer is added to each well of an inert assay microtitre plate e.g. a DYNEX microlite 1 plate.
2.9 100ul of each centrifuged sample and control is removed from the centrifuge plate and dispensed onto the same position on the inert assay microlite plate, bringing the well volume to 120µl.
2.10 The plate is covered with a microtitre plate sealer and incubated at 37°C for 60 minutes.
2.11 The plate is then washed 4 times with Wash Buffer 1.
2.11 200µl of Assay priming Buffer supplied by Enfer Products, Castleblake, Rosegreen Cashel, Co. Tipperary, Ireland, are added to each well of the microtitre plate and the plate incubated for 15 minutes at 37°C.
2.12 The plate is washed 4 times with Wash Buffer 2.

### 3. IMMUNOASSAY

3.1 150µl of primary antibody, rabbit (species) anti-PrP, polyclonal/monoclonal antibody at optimal dilution is dispensed onto the plate.
3.2 The plate is incubated at 37°C for 40 minutes.
3.3 The plate is washed 4 times with Wash Buffer 2.
3.4 150µl of secondary antibody, species-anti-rabbit(species)-HRP at optimal dilution is dispensed onto the plate and the plate incubated at 37°C for 30 minutes. Optimal dilution is determined by a grid assay e.g. plotting varying concentrations of primary and secondary antibody to determine optimal light signal (see below).
3.5 The plate is washed 4 times with Wash Buffer 2.

### 4. DETECTION

4.1 150µl of an enhanced chemiluminescent reagent-Amerlite reagent, supplied by Johnson & Johnson Clinical Diagnostics, UK is added to the plate.
4.2 The plate is incubated at 37°C for 3 minutes.
4.3 The light signal is read using a Labsystems Chemiluminometer, supplied by Medical Supply Company, Dublin, Ireland, which reads the luminescence from each well of the plate by scanning the entire IR-Visible-W spectra and extrapolating results.
4.4 The light signals from the plate are transferred to a customised software package, designed by G.K.S. Software, Dublin Ireland.
4.5 Each light signal is assigned to a corresponding bar-code and a report printed.
4.6 Results are quoted in chemiluminescent light units - L.U.

### SOURCE OF REAGENTS

1. Prion Specific Antibodies - anti-PrP- are supplied by Enfer Scientific Ltd. and are polyclonal antibodies raised to the following synthetic prion peptide:
   PRION SEQUENCE 
N-TERM -

MVKSHIGSWILVLFVVAMWSDVGLCKKRPKPGGGWNTGGSRYPGQ- 44
GSPGGNRYPPQGGGGWGQPHGGGWGQPHGGGWGQPHGGGWGQP-87
GGGGWGQGGSHSQWNKPSKPPKTNMKHVAGAAAAGAVVGGLGGY- 131
MLGSAMSSPLIHFGNDYEDRYTRENMYRYPNQVYYRPVDRYSNQNN-177
 Single Letter Amino Acid Code:
   A -Alanine, C- Cysteine, D -AsparticAcid, E- GlutamicAcid, F- Phenylalanine, G-Glycine, H - Histidine, I - Isoleucine, K - Lysine, L - Leucine, M- Methionine, N-Asparagine, P - Proline, Q - Glutamine, R -Arginine, S- Serine, T- Threonine, V-Valine, W- Tryptophan, Y- Tyrosine.
   This 29 amino acid peptide is used to satisfactorily raise anti-PrP antibodies for the ELISA technique. This sequence was identified by Proteus International UK, using molecular techniques, as being a potentially useful peptide for production of anti-PrP antibodies that could be used successfully for TSE detection. The highlighted sequence is used to raise rabbit anti-PrP antibodies. The peptide (polypeptide, protein) is conjugated to activated ovalbumin, BSA or KLH and injected intra-muscularly or sub-cutaneously in Freund's Complete Adjuvant. Booster injections are sub-cutaneous and Freund's Incomplete Adjuvant is used. The rabbits are bled at 30 and 50 days post immunisation, or until a satisfactory titre is reached. Monoclonal antibodies can then be produced if required.
2. Enfer Buffers 1-3 are produced and supplied by Enfer Scientific.
   Homogenising Buffer 1: 16% Methanol/Ethanol
      15 % Sodium Dodecyl Sulphate
      0.1% Bovine Serum Albumin
      The buffer is made up to the required volume, stirring in 0.1M TRIS/HCL Buffer, final pH of 8.0+0.1.This buffer is stored from 16-25°C with an expiry of 1 year.
   Differential Buffer 2: Proteinase K
   This buffer is made up to the required concentration (0.1 to 10mg/ml) in 0.1M TRIS/HCL. Buffer, final pH of 8 + 0.1. This buffer is stored at -20°C with an expiry of 1 year.
   Assay priming Buffer 3: 3M Guanidine or 6M Urea
      0.1M Sodium Hydroxide 0.1M Sodium Hydroxide
   This buffer is made up to the required concentration in ROH₂O (reverse osmosis distilled water) and allowed to reach room temperature before checking pH, which should preferably be greater than 11. This buffer is stored at 16-25°C with an expiry of 1 year.
3. Wash Buffers 1-2 are produced and supplied by Enfer Scientific.
   Wash Buffer 1: 5% Sodium Chloride
   This buffer is made up to the required volume in ROH₂O. pH irrelevant. This buffer is stored at room temperature with an expiry of 1 year .
   Wash Buffer 2: 0.1M Phosphate Buffered Saline
      0.5% Tween 20
   This buffer is made up to the required volume in ROH₂O with a pH of 7.6 + 0.1.
   This buffer is stored at room temperature with an expiry of 1 year.
4. Chemiluminescent reagent is supplied by Johnson and Johnson, Clinical Diagnostics, UK and results are read using a Labsystems Chemiluminometer, available from Medical Supply Company, Dublin, Ireland.

### Determination of antibody concentration - Grid Assay

Negative and positive controls are put in duplicate into every second set of wells. A series of antibody dilutions are made up 1/500 to 1/2000 as shown in Table 1 and the test is run. At the primary antibody addition stage the dilutions of primary antibody are put onto the negative and positive sample wells. At the end of the test the antibody concentration which gives the highest positive control value, without giving a high negative control result is used.

**Table 1**

| Primary Antibody Conc. | | 1 | 2 | 3 | 4 | 5 | 6 | 7 | 8 | 9 | 10 | 11 | 12 |
|---|---|---|---|---|---|---|---|---|---|---|---|---|---|
| 1/500 | A | Neg | | | | | | | | | | | |
| | B | Pos | | | | | | | | | | | |
| 1/1000 | C | Neg | | | | | | | | | | | |
| | D | Pos | | | | | | | | | | | |
| 1/1500 | E | Neg | | | | | | | | | | | |
| | F | Pos | | | | | | | | | | | |
| 1/2000 | G | Neg | | | | | | | | | | | |
| | H | Pos | | | | | | | | | | | |

### Example 2

The antibody optimisation assay is carried out as follows: positive and negative tissue, confirmed by the Veterinary Research Laboratory is used. This is the same material used for controls in the assay.

The tissue is diluted 1/15 in Homogenising buffer and homogenised for 2 minutes.
200ul of negative homogenate is then placed in rows A, C. E and G
200ul of positive homogenate is placed in rows B, D, F and H.
The plate is centrifuged for 10 mins at 400 rpm.
20ul of differential buffer is added to the assay plate and 100ul of each sample is transferred to corresponding wells.
The plate is then sealed and incubated fro 1 hour at 37°C,
The plate is washed with wash buffer 1 and 200ul of primary buffer is added. This is incubated for 15 minutes at 37°C.

A series of antibody solutions are made up in wash buffer 2, i.e. 1/500, 1/1000, 1/1500 and 1/2000, if the antibody has a higher titre or lower titre than this range a higher or extended range can be tried after the initial assay.

Following incubation in priming buffer the plate is washed with wash buffer 2. 150ul of each antibody concentration is then added to the appropriate wells and incubated for 40 minutes at 37°C. The plate is washed with wash buffer 2 and 150ul of secondary antibody is added to all wells for 30 minutes at 37°C and then the plate is washed again with wash buffer 2 and 150ul of chemiluminesence substrate is added to all wells. After a 3 minute incubation at 37°C the wells are read the chemiluminometer.

The antibody concentration which gives the best result i.e. low negative preferably <1.0, while giving a good positive result eg 500 is the concentration chosen.

As mentioned earlier a higher dilution range can be selected before the antibody is used, but this assay gives a good idea whether or not the antibody can be used.

| | | | | | | | | | | | | | | |
|---|---|---|---|---|---|---|---|---|---|---|---|---|---|---|
| 1/500 | { | N | 4.235 | 3.897 | 3.554 | 4.116 | 2.698 | 3.487 | 4.679 | 3.229 | 4.569 | 3.487 | 3.778 | 4.946 |
| | { | P | 724.5 | 759.4 | 811.6 | 792.4 | 772.5 | 851.3 | 764.2 | 844.2 | 793.4 | 882.4 | 816.7 | 799.2 |
| | | | | | | | | | | | | | | |
| 1/100 | { | N | 2.987 | 2.897 | 2.378 | 2.556 | 2.446 | 2.791 | 3.016 | 1.945 | 2.069 | 2.449 | 2.164 | 2.310 |
| | { | P | 563.4 | 547.2 | 538.0 | 587.1 | 564.2 | 603.7 | 589.4 | 556.1 | 567.3 | 613.7 | 508.6 | 543.7 |
| | | | | | | | | | | | | | | |
| 1/500 | { | N | 1.579 | 0.945 | 1.344 | 1.067 | 1.337 | 1.298 | 1.049 | 0.879 | 1.346 | 1.222 | 1.340 | 1.206 |
| | { | P | 457.6 | 476.5 | 503.0 | 402.1 | 449.5 | 432.8 | 463.7 | 433.0 | 492.7 | 409.7 | 430.9 | 443.1 |
| | | | | | | | | | | | | | | |
| 1/2000 | { | N | 0.861 | 0.936 | 0.727 | 0.914 | 0.866 | 0.569 | 0.612 | 0.493 | 0.721 | 0.642 | 0.463 | 0.762 |
| | { | P | 366.9 | 322.3 | 390.1 | 377.5 | 376.1 | 349.4 | 346.7 | 334.8 | 329.4 | 367.4 | 329.4 | 337.9 |

### Example 3

**Experimental Data** on the suitability of the Enfer TSE assay as a means of detecting TSE in different tissues, namely, central nervous tissues (CNS), lymph tissue and blood and in different species, ovine and bovine.

**Results of routine testing using the TSE Assay of the invention in 2001.**

### Bovine CNS tissue

| | |
|---|---|
| Total samples tested : | 664942 |
| No. of positives detected: | 134 |
| No. of positives confirmed : | 134 |
| by immunohistochemistry | |

### Ovine CNS tissue

| | |
|---|---|
| Total samples tested : | 16467 |
| No. of positives detected : | 60 |
| No. of positives confirmed : | 60 |
| by immunohistochemistry | |

### Ovine Lymph tissue

| | |
|---|---|
| Total samples tested : | 6974 |
| No. of positives detected: | 10 |
| Confirmed : | 9* |
| by immunohistochemistry | |

It was not possible to confirm one lymph tissue sample. This tissue was from a 14 week old lamb and was in the very early stages of infection. This may explain the difficulties with confirmation.

### Ovine Blood tissue (1998)

| | |
|---|---|
| Total tested : | 3 |
| No. of positives: | 3 |

This was fresh blood derived from clinical Scrapie cases. All cases were found to be weakly positive and no confirmatory method exists to do a follow up analysis.

## Claims

1. A method of detecting TSE comprising (a) treating a sample of tissue, blood or a blood derivative from a test subject with a homogenization buffer comprising alcohol in an amount up to 30% and between 5% and 30% detergent and dispensing the sample onto an inert support, (b) adding an agent which degrades normal prion protein, (c) adding an agent which denatures abnormal prion protein, (d) adding a prion-specific antibody, and (e) detecting binding of the antibody to the sample.

2. A method as claimed in claim 1 wherein step (a) further comprises the addition of a protein.

3. A method as claimed in claim 2 wherein the protein is a high molecular weight protein, selected from the group consisting of bovine serum albumin, ovalbumin, normal serum of animal origin, an albumin substitute.

4. A method as claimed in claim 2 or 3 wherein the protein is formulated in a buffer containing up to 5%, preferably 0.05 to 0.4% protein.

5. A method as claimed in any preceding claim wherein the alcohol used in step (a) is a C₁ to C₂₄ alcohol, preferably a C₁ to C₅ alcohol.

6. A method as claimed in claim 5 wherein the alcohol is selected from methanol, ethanol, propanol, butanol, pentanol, hexanol, heptanol, octanol, nonanol, decanol, undecanol, dodecanol, tridecanol, , pantadecanol, cetyl alcohol, heptadecanol, stearyl alcohol, nonadecanol, eicosanol, docasanol, dodecyl alcohol, lauryl alcohol, octadecanol, preferably methanol or ethanol.

7. A method as claimed in any preceding claim wherein the alcohol is formulated in a buffer containing 10% to 20% (by weight), preferably 8 to 12% alcohol.

8. A method as claimed in any preceding claim wherein the detergent of step (a) is selected from sodium dodecyl sulphate, sarcosyl, lithium dodecyl sulphate, cholic acid, deoxycholic acid, Triton X-100, octanoic acid, Brij (trade mark) 35 solution, N-Cetyl-N,N,N-trimethylammoniumbromide, CHAPS, CHAPSO, Digitonin, nonanoyl-N-methylglucamide, n-Octyl-B-D-glucopyranoside, alkyl sodium sulphate, dioctyl sodium sulfosuccinate, sodium oleate, sodium lauryl sulphate, 1,2,3-Octanetriol, 3-Diethylamino-1 propyne, Chondroitinase ABC, sodium Ionophore 1, most preferably sodium dodecyl sulphate.

9. A method as claimed in any preceding claim wherein the homogenization buffer contains 10 to 20% detergent.

10. A method as claimed in any preceding claim wherein in step (b) the agent which degrades normal prion protein is a protease.

11. A method as claimed in claim 10 wherein the protease is a broad spectrum protease, preferably selected from proteinase K, subtilisin Carlsberg, pepsin.

12. A method as claimed in wherein claim 10 wherein the protease is formulated in a buffer containing up to 10mg/ml, preferably 0.05mg/ml to 4mg/ml, more preferably 0.2mg/ml to 0.6mg/ml protein.

13. A method as claimed in any preceding claim wherein the agent which denatures abnormal prion protein in step (c) is a denaturing salt solution.

14. A method as claimed in claim 13 wherein the agent is selected from guanidine, urea, and a standard denaturing solution.

15. A method as claimed in any preceding claim wherein the denaturing agent further includes up to 0.5M sodium hydroxide.

16. A method as claimed in any preceding claim wherein the denaturing salt is formulated in a buffer containing 0.1 to 10M, preferably 2 to 8M denaturing salt.

17. A method as claimed in any preceding claim wherein the buffer of step (a) (homogenising buffer) is made up to have a final pH of 5.0 to 11.0, preferably pH 7.0 to 9.0, more preferably pH 7.5 to 8.5.

18. A method as claimed in any preceding claim wherein the buffer of step (b) (differential buffer) is made up to a final pH of 2.0 to 11.0, preferably pH 2.0 to 8.5, more preferably 7.5 to 8.5.

19. A method as claimed in any preceding claim wherein the buffer of step (c) (assay priming buffer) has a pH of 1 to 14, preferably 9 to 12, more preferably at least 11.

20. A method as claimed in any preceding claim wherein following step (b) the reaction mixture is washed with a first wash buffer comprising up to 10%, preferably 4 to 6% buffered solution.

21. A method as claimed in any preceding claim wherein the assay priming buffer is allowed to incubate with the reaction mixture for about 5 to 30 minutes, preferably 10 to 20 minutes.

22. A method as claimed in any preceding claim wherein after treatment with the assay priming buffer the mixture is washed with a second wash buffer having a final pH of 5.0 to 11.0, preferably pH 7.0 to 8.0.

23. A method as claimed in claim 20 wherein the first wash buffer comprises sodium chloride, calcium chloride, and/or zinc chloride.

24. A method as claimed in claim 22 wherein the second wash buffer comprises phosphate buffered saline with the optional addition of 0 to 5% Tween 20.

25. A method as claimed in any preceding claim wherein the prion specific antibody is raised to a prion sequence which is to the 5' side of amino-acid number 60 of the prion sequence.

26. A method as claimed in any one of the preceding claim comprising reacting a sample of blood or a blood derivative or tissue obtained from a test subject with a prion specific antibody and detecting the binding of the antibody to the sample by standard immunoassay, wherein the tissue, blood or blood derivative sample is mixed prior to addition of the antibody with a first buffer comprising 0 to 30% of C₁ to C₅ alcohol, 5 to 30% sodium dodecyl sulphate and 0 to 5% bovine serum albumin made up to volume in about 0.1 M TRIS/HCL or phosphate buffer, the buffer having a final pH of 7.0 to 9.0.

27. A method of detecting Transmissable Spongiform Encephalopathies comprising homogenizing a sample of blood or blood derivative or tissue obtained from a test subject in a homogenization buffer comprising alcohol in an amount up to 30% and between 5% and 30% detergent, reacting the tissue, blood or blood derivative sample with a prion specific antibody and detecting the binding of the antibody to the sample by standard immunoassay, wherein the blood or tissue sample is dispensed onto an inert support and incubated with a buffer comprising up to 10 mg/ml of a broad spectrum protease enzyme in a buffer with a final pH of 2.0 to 11.0 prior to reaction with the antibody.

28. A method of detecting Transmissable Spongiform Encephalopathies comprising homogenizing a sample of blood or a blood derivative or tissue obtained from a test subject in a homogenization buffer comprising alcohol in an amount up to 30% and between 5% and 30% detergent, reacting the tissue, blood or blood derivative sample with a prion specific antibody and detecting the binding of the antibody to the sample by standard immunoassay, wherein the sample is incubated with a buffer comprising 0.1 to 10M denaturing solution, preferably 3 to 6M Guanidine or 2 to 8M urea, and up to 0.5M sodium hydroxide made up in reverse osmosis distilled water, with a pH of at least 11 at room temperature, prior to reaction with the antibody.

## Patentansprüche

1. Verfahren zum Nachweisen von TSE, umfassend (a) Behandeln einer Gewebs-, Blut- oder Blutderivatprobe von einem Testsubjekt mit einem Homogenisationspuffer, der Alkohol in einer Menge von bis zu 30% und zwischen 5% and 30% Detergens umfasst, und Abgeben der Probe auf einen inerten Träger, (b) Zugeben eines Agens, das normales Prionprotein abbaut, (c) Zugeben eines Agens, das anomales Prionprotein denaturiert, (d) Zugeben eines Prion-spezifischen Antikörpers, und (e) Nachweisen der Bindung des Antikörpers an die Probe.

2. Verfahren nach Anspruch 1, wobei Schritt (a) ferner die Zugabe eines Proteins umfasst.

3. Verfahren nach Anspruch 2, wobei das Protein ein Protein mit hohem Molekulargewicht ausgewählt aus der Gruppe bestehend aus bovinem Serumalbumin, Ovalbumin, normalem Serum tierischer Herkunft und einem Albuminersatz ist.

4. Verfahren nach Anspruch 2 oder 3, wobei das Protein in einem Puffer formuliert ist, der bis zu 5%, vorzugsweise 0,05 bis 0,4%, Protein enthält.

5. Verfahren nach einem der vorhergehenden Ansprüche, wobei der in Schritt (a) verwendete Alkohol ein C₁ bis C₂₄ Alkohol, vorzugsweise ein C₁ bis C₅ Alkohol, ist.

6. Verfahren nach Anspruch 5, wobei der Alkohol aus Methanol, Ethanol, Propanol, Butanol, Pentanol, Hexanol, Heptanol, Octanol, Nonanol, Decanol, Undecanol, Dodecanol, Tridecanol, Pantadecanol, Cetylalkohol, Heptadecanol, Stearylalkohol, Nonadecanol, Eicosanol, Docasanol, Dodecylalkohol, Laurylalkohol, Octadecanol, vorzugsweise aus Methanol oder Ethanol, ausgewählt ist.

7. Verfahren nach einem der vorhergehenden Ansprüche, wobei der Alkohol in einem Puffer formuliert ist, der 10% bis 20% (Gew.-%), vorzugsweise 8 bis 12%, Alkohol enthält.

8. Verfahren nach einem der vorhergehenden Ansprüche, wobei das Detergens des Schritts (a) aus Natriumdodecylsulfat, Sarkosyl, Lithiumdodecylsulfat, Cholsäure, Desoxycholsäure, Triton X-100, Octansäure, Brij (Marke) 35-Lösung, N-Cetyl-N,N,N-trimethylammoniumbromid, CHAPS, CHAPSO, Digitonin, Nonaroyl-N-methylglucamid, N-Octyl-β-D-glucopyranosid, Alkylnatriumsulfat, Dioctylnatriumsulfosuccinat, Natriumoleat, Natriumlaurylsulfat, 1,2,3-Octantriol, 3-Diethylamino-1-propin, Chondroitinase ABC, Natrium-Ionophor 1, besonders bevorzugt Natriumdodecylsulfat, ausgewählt ist.

9. Verfahren nach einem der vorhergehenden Ansprüche, wobei der Homogenisationspuffer 10 bis 20% Detergens enthält.

10. Verfahren nach einem der vorhergehenden Ansprüche, wobei in Schritt (b) das Agens, das normales Prionprotein abbaut, eine Protease ist.

11. Verfahren nach Anspruch 10, wobei die Protease eine Breitbandprotease ist, die vorzugsweise aus Proteinase K, Subtilisin Carlsberg, Pepsin ausgewählt ist.

12. Verfahren nach Anspruch 10, wobei die Protease in einem Puffer formuliert ist, der bis zu 10 mg/ml, vorzugsweise 0,05 mg/ml bis 4 mg/ml, bevorzugter 0,2 mg/ml bis 0,6 mg/ml, Protein enthält.

13. Verfahren nach einem der vorhergehenden Ansprüche, wobei das Agens, das anomales Prionprotein in Schritt (c) denaturiert, eine denaturierende Salzlösung ist.

14. Verfahren nach Anspruch 13, wobei das Agens aus Guanidin, Harnstoff und einer Standarddenaturierungslösung ausgewählt ist.

15. Verfahren nach einem der vorhergehenden Ansprüche, wobei das Denaturierungsagens ferner bis zu 0,5 M Natriumhydroxid umfasst.

16. Verfahren nach einem der vorhergehenden Ansprüche, wobei das denaturierende Salz in einem Puffer formuliert ist, der 0,1 bis 10 M, vorzugsweise 2 bis 8 M, denaturierendes Salz enthält.

17. Verfahren nach einem der vorhergehenden Ansprüche, wobei der Puffer des Schritts (a) (Homogenisationspuffer) so zusammengesetzt ist, dass dieser einen End-pH von 5,0 bis 11,0, vorzugsweise pH 7,0 bis 9,0, bevorzugter pH 7,5 bis 8,5, aufweist.

18. Verfahren nach einem der vorhergehenden Ansprüche, wobei der Puffer des Schritts (b) (Differenzialpuffer) so zusammengesetzt ist, dass dieser einen End-pH von 2,0 bis 11,0, vorzugsweise pH 2,0 bis 8,5, bevorzugter 7,5 bis 8,5, aufweist.

19. Verfahren nach einem der vorhergehenden Ansprüche, wobei der Puffer des Schritts (c) (Assay-Grundpuffer) einen pH von 1 bis 14, vorzugsweise 9 bis 12, bevorzugter wenigstens 11, aufweist.

20. Verfahren nach einem der vorhergehenden Ansprüche, wobei nach dem Schritt (b) die Reaktionsmischung mit einem ersten Waschpuffer, der bis zu 10%, vorzugsweise 4 bis 6%, einer gepufferten Lösung umfasst, gewaschen wird.

21. Verfahren nach einem der vorhergehenden Ansprüche, wobei der Assay-Grundpuffer mit der Reaktionsmischung für etwa 5 bis 30 Minuten, vorzugsweise 10 bis 20 Minuten, inkubieren gelassen wird.

22. Verfahren nach einem der vorhergehenden Ansprüche, wobei die Mischung nach Behandlung mit dem Assay-Grundpuffer mit einem zweiten Waschpuffer mit einem End-pH von 5,0 bis 11,0, vorzugsweise pH 7,0 bis 8,0, gewaschen wird.

23. Verfahren nach Anspruch 20, wobei der erste Waschpuffer Natriumchlorid, Calciumchlorid und/oder Zinkchlorid umfasst.

24. Verfahren nach Anspruch 22, wobei der zweite Waschpuffer phosphatgepufferte Salzlösung mit der optionalen Zugabe von 0 bis 5% Tween 20 umfasst.

25. Verfahren nach einem der vorhergehenden Ansprüche, wobei der Prionspezifische Antikörper gegen eine Prionsequenz gerichtet ist, die sich auf der 5'- Seite der Aminosäurenummer 60 der Prionsequenz befindet.

26. Verfahren nach einem der vorhergehenden Ansprüche, umfassend das Reagieren einer von einem Testsubjekt erhaltenen Blut- oder Blutderivat- oder Gewebsprobe mit einem Prion-spezifischen Antikörper und Nachweisen der Bindung des Antikörpers an die Probe durch einen Standard-Immunoassay, wobei die Gewebs-, Blut- oder Blutderivatprobe vor Zugabe des Antikörpers mit einem ersten Puffer gemischt wird, umfassend 0 bis 30% C₁ bis C₅ Alkohol, 5 bis 30% Natriumdodecylsulfat und 0 bis 5% bovines Serumalbumin, aufgefüllt mit etwa 0,1 M TRIS/HCI oder Phosphatpuffer, wobei der Puffer einen End-pH von 7,0 bis 9,0 aufweist.

27. Verfahren zum Nachweisen von übertragbaren spongioformen Enzephalopathien, umfassend das Homogenisieren einer von einem Testsubjekt erhaltenen Blut- oder Blutderivat- oder Gewebsprobe in einem Homogenisationspuffer, umfassend Alkohol in einer Menge bis zu 30% und zwischen 5% und 30% Detergens, das Reagieren der Gewebs-, Blut- oder Blutderivatprobe mit einem Prion-spezifischen Antikörper und das Nachweisen der Bindung des Antikörpers an die Probe durch einen Standard-Immunoassay, wobei die Blut- oder Gewebsprobe auf einen inerten Träger abgegeben und vor der Reaktion mit dem Antikörper mit einem Puffer inkubiert wird, der bis zu 10 mg/ml eines Breitbandproteaseenzyms in einem Puffer mit einem End-pH von 2,0 bis 11,0 umfasst.

28. Verfahren zum Nachweisen von übertragbaren spongioformen Enzephalopathien, umfassend das Homogenisieren einer von einem Testsubjekt erhaltenen Blut- oder Blutderivat- oder Gewebsprobe in einem Homogenisationspuffer, umfassend Alkohol in einer Menge bis zu 30% und zwischen 5% und 30% Detergens, das Reagieren der Gewebs-, Blut- oder Blutderivatprobe mit einem Prion-spezifischen Antikörper und das Nachweisen der Bindung des Antikörpers an die Probe durch einen Standard-Immunoassay, wobei die Probe vor der Reaktion mit dem Antikörper mit einem Puffer, umfassend 0,1 bis 10 M denaturierende Lösung, vorzugsweise 3 bis 6 M Guanidin oder 2 bis 8 M Harnstoff, und bis zu 0,5 M Natriumhydroxid, hergestellt in destilliertem Umkehrosmosewasser, mit einem pH von wenigstens 11 bei Raumtemperatur inkubiert wird.

## Revendications

1. Procédé de détection de l'EST comprenant (a) le traitement d'un échantillon de tissu, de sang ou d'un dérivé sanguin provenant d'un sujet de test, avec un tampon d'homogénéisation comprenant de l'alcool en une quantité allant jusqu'à 30 % et entre 5 % et 30 % de détergent et le placement de l'échantillon sur un support inerte, (b) l'addition d'un agent qui décompose une protéine de prion normale, (c) l'addition d'un agent qui dénature une protéine de prion anormale, (d) l'addition d'un anticorps spécifique du prion, et (e) la détection de la liaison de l'anticorps à l'échantillon.

2. Procédé selon la revendication 1, dans lequel l'étape (a) comprend en outre l'addition d'une protéine.

3. Procédé selon la revendication 2, dans lequel la protéine est une protéine de haut poids moléculaire choisie dans le groupe comprenant la sérumalbumine bovine, l'ovalbumine, le sérum normal d'origine animale, un substitut d'albumine.

4. Procédé selon la revendication 2 ou 3, dans lequel la protéine est formulée dans un tampon contenant jusqu'à 5 %, de préférence 0,05 à 0,4 % de protéine.

5. Procédé selon l'une quelconque des revendications précédentes, dans lequel l'alcool utilisé à l'étape (a) est un alcool en C₁ à C₂₄, de préférence, un alcool en C₁ à C₆.

6. Procédé selon la revendication 5, dans lequel l'alcool est choisi parmi le méthanol, l'éthanol, le propanol, le butanol, le pentanol, l'hexanol, l'heptanol, l'octanol, le nonanol, le décanol, l'undécanol, le dodécanol, le tridécanol, le pentadécanol, l'alcool de cétyle, l'heptadécanol, l'alcool de stéaryle, le nonadécanol, l'eicosanol, le docasanol, l'alcool de dodécyle, l'alcool de lauryle, le cétadécanol, de préférence le méthanol ou l'éthanol.

7. Procédé selon l'une quelconque des revendications précédentes, dans lequel l'alcool est formulé dans un tampon contenant 10 % à 20 % (en poids), de préférence, 8 à 12 % d'alcool.

8. Procédé selon l'une quelconque des revendications précédentes, dans lequel le détergent de l'étape (a) est choisi parmi le dodécyl-sulfate de sodium, le sarcosyle, le dodécyl-sulfate de lithium, l'acide cholique, l'acide désoxycholique, le Triton X-100, l'acide octanoïque, une solution de Brij 35 (marque déposée), le bromure de N-cétyl-N,N,N-triméthylammonium, le CHAPS, le CHAPSO, la digitonine, le nonanoyl-N-méthylglucamide, le n-octyl-B-D-glucopyranoside, le sulfate d'alkyle sodium, le sulfosuccinate de dioctyle sodium, l'oléate de sodium, le lauryl-sulfate de sodium, le 1,2,3-octanetriol, le 3-diéthylamino-1-propyne, la chondroïtinase ABC, l'ionophore de sodium 1, de manière préférée entre toutes, le dodécyl-sulfate de sodium.

9. Procédé selon l'une quelconque des revendications précédentes, dans lequel le tampon d'homogénéisation contient 10 à 20 % de détergent.

10. Procédé selon l'une quelconque des revendications précédentes, dans lequel à l'étape (b), l'agent qui décompose la protéine de prion normale est une protéase.

11. Procédé selon la revendication 10, dans lequel la protéase est une protéase à large spectre, choisie de préférence parmi la protéinase K, la subtilisine Carlsberg, la pepsine.

12. Procédé selon la revendication 10, dans lequel la protéase est formulée dans un tampon contenant jusqu'à 10 mg/ml, de préférence 0,05 mg/ml à 4 mg/ml, de manière davantage préférée, 0,2 mg/ml à 0,6 mg/ml de protéine.

13. Procédé selon l'une quelconque des revendications précédentes, dans lequel l'agent qui dénature la protéine de prion anormale de l'étape (c) est une solution physiologique dénaturante.

14. Procédé selon la revendication 13, dans lequel l'agent est choisi parmi la guanidine, l'urée et une solution dénaturante standard.

15. Procédé selon l'une quelconque des revendications précédentes, dans lequel l'agent dénaturant comprend en outre jusqu'à 0,5 M d'hydroxyde de sodium.

16. Procédé selon l'une quelconque des revendications précédentes, dans lequel le sel dénaturant est formulé dans un tampon contenant 0,1 à 10 M, de préférence, 2 à 8 M de sel dénaturant.

17. Procédé selon l'une quelconque des revendications précédentes, dans lequel le tampon de l'étape (a) (tampon d'homogénéisation) est constitué de façon à présenter un pH final de 5,0 à 11,0, de préférence un pH de 7,0 à 9,0, de manière davantage préférée, un pH de 7,5 à 8,5.

18. Procédé selon l'une quelconque des revendications précédentes, dans lequel le tampon de l'étape (b) (tampon différentiel) est constitué de façon à présenter un pH final de 2,0 à 11,0, de préférence, de 2,0 à 8,5, de manière davantage préférée, de 7,5 à 8,5.

19. Procédé selon l'une quelconque des revendications précédentes, dans lequel le tampon de l'étape (c) (tampon d'amorçage de l'essai) a un pH de 1 à 14, de préférence, de 9 à 12, de manière davantage préférée, d'au moins 11.

20. Procédé selon l'une quelconque des revendications précédentes, dans lequel après l'étape (b), le mélange réactionnel est lavé avec un premier tampon de lavage comprenant jusqu'à 10 %, de préférence 4 à 6 % de solution tamponnée.

21. Procédé selon l'une quelconque des revendications précédentes, dans lequel on laisse le tampon d'amorçage de l'essai incuber avec le mélange réactionnel pendant environ 5 à 30 minutes, de préférence, 10 à 20 minutes.

22. Procédé selon l'une quelconque des revendications précédentes, dans lequel après le traitement avec le tampon d'amorçage de l'essai, le mélange est lavé avec un deuxième tampon de lavage ayant un pH final de 5,0 à 11, 0, de préférence, un pH de 7,0 à 8,0.

23. Procédé selon la revendication 20, dans lequel le premier tampon de lavage comprend du chlorure de sodium, du chlorure de calcium et/ou du chlorure de zinc.

24. Procédé selon la revendication 22, dans lequel le deuxième tampon de lavage comprenant une solution physiologique tamponnée au phosphate avec l'addition éventuelle de 0 à 5 % de Tween 20.

25. Procédé selon l'une quelconque des revendications précédentes, dans lequel l'anticorps spécifique du prion est dirigé contre une séquence de prion qui est du côté 5' de l'acide aminé numéro 60 de la séquence de prion.

26. Procédé selon l'une quelconque des revendications précédentes, comprenant la réaction d'un échantillon de sang ou d'un dérivé sanguin ou d'un tissu obtenu chez un sujet de test avec un anticorps spécifique du prion et la détection de la liaison de l'anticorps à l'échantillon par un essai immunologique standard, le tissu, le sang ou le dérivé sanguin étant mélangé, avant l'addition de l'anticorps, avec un premier tampon comprenant 0 à 30 % d'alcool en C₁ à C₅, 5 à 30 % de dodécyl-sulfate de sodium et 0 à 5 % de sérumalbumine bovine constituant un volume dans du TRIS/HCL à environ 0,1 M ou un tampon de phosphate, le tampon ayant un pH final de 7,0 à 9,0.

27. Procédé de détection des encéphalopathies spongiformes transmissibles comprenant l'homogénéisation d'un échantillon de sang ou d'un dérivé sanguin ou d'un tissu obtenu chez un sujet de test dans un tampon d'homogénéisation comprenant de l'alcool en une quantité allant jusqu'à 30 % et entre 5 % et 30 % de détergent, la réaction du tissu, du sang ou du dérivé sanguin avec un anticorps spécifique du prion et la détection de la liaison de l'anticorps à l'échantillon par un essai immunologique standard, l'échantillon de sang ou de tissu étant placé sur un support inerte et incubé avec un tampon comprenant jusqu'à 10 mg/ml d'une enzyme protéase à large spectre dans un tampon présentant un pH final de 2,0 à 11,0 avant la réaction avec l'anticorps.

28. Procédé de détection des encéphalopathies spongiformes transmissibles comprenant l'homogénéisation d'un échantillon de sang ou d'un dérivé sanguin ou d'un tissu obtenu chez un sujet de test dans un tampon d'homogénéisation comprenant de l'alcool en une quantité allant jusqu'à 30 % et entre 5 % et 30 % de détergent, la réaction du tissu, du sang ou du dérivé sanguin avec un anticorps spécifique du prion et la détection de la liaison de l'anticorps à l'échantillon par un essai immunologique standard, l'échantillon étant incubé avec un tampon comprenant une solution dénaturante 0,1 à 10 M, de préférence de guanidine 3 à 6 M ou d'urée 2 à 8 M et une solution d'hydroxyde de sodium jusqu'à 0,5 M constituée dans de l'eau distillée par osmose inverse, présentant un pH d'au moins 11 à température ambiante, avant la réaction avec l'anticorps.
